# FASCICULE DE BREVET EUROPEEN

(11) **EP 0 488 869 B1**
(45) Date de publication et mention de la délivrance du brevet: **07.02.1996**
(21) Numéro de dépôt: 91403175.2
(22) Date de dépôt: 25.11.1991
(51) Int. Cl.: C07D 213/73, G02F 1/35

(54) **Sels de 2-amino-5-nitropyridinium, utilisables en optique non-linéaire et en électro-optique et leur procédé de préparation**
Salze von 2-amino-5-nitropyridinium für die nichtlineare Optik und die Elektro-Optik und Verfahren zu ihrer Herstellung
Salts of 2-amino-5-nitropyridinium for non-linear and electro-optic use, and a process for their preparation

(30) Priorité: 26.11.1990 FR 9014743
(43) Date de publication de la demande: 03.06.1992
(73) Titulaire: FRANCE TELECOM, F-92131 Issy les Moulineaux (FR); CENTRE NATIONAL DE LA RECHERCHE SCIENTIFIQUE (CNRS), F-75700 Paris Cedex 07 (FR)
(72) Inventeur: Masse, René, F-38000 Grenoble (FR); Zyss, Joseph, F-92330 Sceaux (FR)
(74) Mandataire: Des Termes, Monique

(56) Documents cités:
- CHEMICAL ABSTRACTS, vol. 99, no. 24, 1983, page 516, abrégé no. 203179g, Columbus, Ohio, US; M. PERRIN et al.: "Organic materials for nonlinear optics", PROC. SPIE-INT. SOC. OPT. ENG. 1983, 400(NEW OPT. MATER.), 176-82
- JOURNAL OF THE CHEMICAL SOCIETY, CHEMICAL COMMUNICATIONS, no 23, 1989, pages 1856-1859; C.B. AAKERÖY et al.: "A novel class of salts for second harmonic generation"
- CHEMICAL ABSTRACTS, vol. 112, no. 26, 1990, page 488, abrégé no. 242602w, Columbus, Ohio, US; L.T. CHENG et al.: "Nonresonant EFISH and THG studies of nonlinear optical property and molecular structure relations of benzene, stilbene, and other arene derivatives", & PROC. SPIE-INT. SOC. OPT. ENG. 1989 (PUB. 1990)

## Description

La présente invention a pour objet de nouveaux sels de 2-amino-5-nitropyridinium utilisables en optique non-linéaire et en électro-optique, et leur procédé de préparation.

Par le terme "optique non-linéaire", on entend le domaine de l'optique qui s'étend de la conversion des fréquences optiques (obtention d'un rayonnement optique à partir de deux rayonnements de fréquences différentes, la fréquence du rayonnement de conversion étant égale à la somme ou à la différence des fréquences des deux rayonnements ou inversement décomposition d'un faisceau incident en deux faisceaux de fréquences inférieures) à la modulation électro-optique (modification d'une des caractéristiques d'un rayonnement par application d'un champ électrique à un cristal traversé par ledit rayonnement).

On connaît déjà de nombreux matériaux susceptibles de convenir à la conversion des fréquences optiques ou à la modulation électro-optique. Parmi ceux-ci, on peut citer des matériaux minéraux tels que le diphosphate de potassium (KDP), le niobate de lithium et le potassium titane oxyde monophosphate (KTP).

Cependant, ces matériaux minéraux présentent l'inconvénient de manquer d'efficacité, ce qui oblige à les employer sous de fortes épaisseurs. Aussi, depuis quelques années, les recherches ont porté sur la réalisation de cristaux de molécules organiques présentant une efficacité améliorée par rapport aux cristaux inorganiques de diphosphate de potassium ou de niobate de lithium. Ainsi, comme il est décrit par J. Zyss dans Current Trends in Optics, Taylor & Francis, London, 1981, p. 122-134, on a trouvé que les dérivés d'aniline ou de pyridine-N-oxyde comme la métanitroaniline (mNA), la 2-méthyl-4-nitroaniline (MNA), le 2(2,4-dinitrophényl)-aminopropanoate de méthyle (MAP) et la 3-méthyl-4-nitropyridine-1-oxyde (POM) faisant l'objet du FR-A-2 472 201 pouvaient être utilisés dans ce but.

Dans le document EP-A- 0 091 838 on a aussi décrit les propriétés intéressantes du N-(4-nitrophényl)-L-prolinol éventuellement deutéré (NPP).

Récemment R. Masse et A. Durif ont proposé dans Zeitschrift für Kristallogr. 190, 1990, p. 19-32 de nouveaux cristaux piézoélectriques et ferroélectriques, utilisables en optique non-linéaire, constitués par des monophosphates diacides de méthylalaninium. Ces monophosphates sont des matériaux polaires à charpente anionique minérale, utilisant le polyanion (H₂PO₄⁻)ₙ sous la forme de couches à l'intérieur desquelles sont disposés des cations organiques.

La recherche de sels pour l'optique non-linéaire quadratique, dans lesquels des cations présentant un caractère de transfert de charge intramoléculaire et de conjugaison sont associés à des anions organiques chiraux, achiraux ou minéraux, a été aussi développée par G.R. Meredith (ACS Symposium Series 233, 1983, p. 27-56). Dans ce cas, le but est d'obtenir
- un empilement non centrosymétrique de cations organiques achiraux dans une structure cristalline, et
- une orientation optimale de ces cations (axe de transfert de charge) par rapport aux axes diélectriques du cristal, permettant d'assurer la biréfringence nécessaire à l'accord de phases entre les faisceaux interagissants, pour chaque classe de symétrie selon J. Zyss et J.L. Oudar (Pys. Rev.A 26, 2028, 1982).

Ainsi, deux grandes familles de matériaux ont fait l'objet de recherches intensives menées jusqu'ici indépendamment : les monocristaux minéraux, d'une part, et les cristaux moléculaires organiques, d'autre part.

Chacune de ces familles présente des avantages et des inconvénients liés à des paramètres tels que
- l'efficacité non-linéaire (facteur de mérite paramétrique ou électro-optique),
- l'étendue de la plage de transparence,
- le seuil de dommages optiques (sous irradiation continue ou impulsionnelle),
- les qualités mécaniques (dureté, robustesse, tendance au clivage, densité de dislocations, aptitude à la découpe et au polissage),
- la stabilité chimique ou physicochimique (photoréactivité, stabilité à l'air, hygroscopie, tendance à la sublimation), et
- l'aptitude à la cristallogénèse (solubilité, stabilité à la fusion, pureté initiale).

A l'égard de ces différents paramètres, aucune des deux familles ne donne complètement satisfaction. Certains matériaux organiques cristallins se recommandent par leurs facteurs de mérite (gain paramétrique ou tension demi-onde) nettement plus élevés que ceux de leurs homologues minéraux. C'est ainsi que le N-(4-nitrophényl)-L-prolinol (NPP) possède un gain paramétrique supérieur de deux ordres de grandeur à celui du potassium titane oxyde monophosphate (KTP). Cependant, les oxydes minéraux tels que KTP paraissent mieux résister à l'irradiation laser en flux continu.

Les matériaux organiques étudiés à ce jour paraissent en général présenter un optimum du compromis efficacité-transparence plutôt centré dans le proche infrarouge. Cependant, la cohésion des cages minérales ioniques est supérieure à celle des cristaux moléculaires associés par des forces de Van Der Waals moins énergétiques. On peut donc s'attendre à de meilleures propriétés mécaniques. De plus, la croissance en solution aqueuse de volumineux cristaux minéraux, par exemple de monophosphate diacide de potassium (KDP) est maintenant une réalité industrielle bien établie.

Aussi, la présente invention a pour objet de nouveaux sels de cations organiques présentant les avantages des deux types de matériaux, soit d'une part, les polarisabilités élevées des molécules organiques et la possibilité, par le jeu des substitutions chimiques, d'induire des susceptibilités non-linéaires élevées et, d'autre part, la cohésion et la robustesse des empilements des cages anioniques des oxydes minéraux.

Selon l'invention, ces nouveaux sels sont des 2-amino-5-nitropyridinium répondant à la formule : 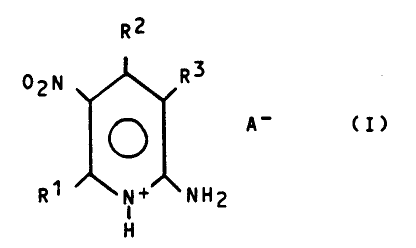
dans laquelle A⁻ représente un anion inorganique choisi parmi H₂PO₄⁻, HSO₄⁻ et H₂AsO₄⁻, et R¹ à R³ qui peuvent être identiques ou différents, représentent H ou CH₃.

Ces nouveaux sels peuvent aussi être partiellement ou totalement deutérés. Dans ce cas, l'un au moins des atomes d'hydrogène de la formule (I) précitée est remplacé par un atome de deutérium.

A titre d'exemple d'un sel conforme à l'invention, on peut citer le monophosphate diacide de 2-amino-5-nitropyridinium, répondant à la formule (Ia) 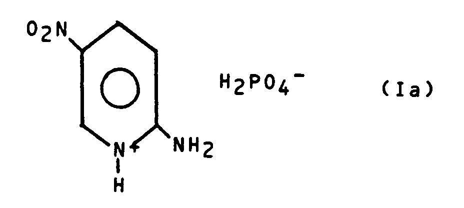
ainsi que les dérivés partiellement ou totalement deutérés de ce sel.

Les sels organo-minéraux de l'invention sont très intéressants en raison de leur structure particulière dans laquelle les cations organiques porteurs d'un moment dipolaire important et dont le spectre comporte un état à transfert de charge intramoléculaire prédominant dans le visible ou le proche infrarouge, sont ancrés par des liaisons hydrogène entre les couches de polyanions minéraux (A⁻)ₙ tels que (H₂PO₄⁻)ₙ.

En effet, dans l'invention, le choix d'un cation 2-amino-5-nitropyridinium, soit d'un dérivé pyridinique de la paranitroaniline, obtenu en remplaçant le groupement CH en position "ortho" du groupement amino donneur d'électrons de la p-nitroaniline par un azote, permet de réaliser l'ancrage et l'insertion du cation par une liaison hydrogène sur les anions minéraux par l'intermédiaire d'un proton attiré sur le même site. Ainsi, on passe d'une entité classique en optique non-linéaire, la paranitroaniline, à une molécule également efficace en optique non-linéaire, plus transparente et susceptible de se lier aisément par l'intermédiaire de liaisons hydrogènes à une chaîne d'anions minéraux.

La molécule de 2-amino-5-nitropyridine qui est un composé neutre, était connue antérieurement mais la structure centrosymétrique du cristal moléculaire correspondant en interdit toute utilisation en optique non-linéaire pour les effets du second ordre.

Selon l'invention, la complexation, dans un réseau cristallin ionique, de cette molécule avec une chaîne polyanionique permet d'aboutir à une efficacité non linéaire bien supérieure à celle des cristaux organominéraux connus tels que les composés décrits par AAKEROY et al dans Journal of the Chemical Society Chemical Communications, n° 23, 1989, p. 1857 dont l'efficacité est inférieure à celle de l'urée puisque le meilleur composé ne dépasse pas cinq fois le quartz.

Dans l'invention, le choix de l'anion minéral, H₂PO₄⁻, HSO₄⁻ ou H₂ASO₄⁻ permet de tirer profit de la modularité stérique et chimique de ces édifices ioniques minéraux, et de leur stabilité pour former un sous-réseau inorganique stable et structurant, apte à fixer et éventuellement à orienter et polariser les cations 2-amino-5-nitropyridinium.

De plus, grâce à ces deux choix, on a une compatibilité chimique entre les unités organique et minérale du composé et une miscibilité à forte concentration organique ; on maintient ou on améliore les propriétés électroniques sous-jacentes à la non-linéarité de la paranitroaniline (transparence, conjugaison, transfert de charge) ; et on obtient un composé capable d'être mis facilement sous la forme de monocristaux, ayant une structure non centrée.

Ainsi, dans le sel de l'invention, l'empilement des cations organiques achiraux est déterminé par la structure bidimensionnelle du sous-réseau des anions minéraux, constitué par exemple par des unités H₂PO₄⁻ fortement dipolaires. Ce sous réseau possède une cohésion spécifique due à des liaisons hydrogènes courtes, que ne sauraient compromettre les cations organiques associés (structure "guest/host") et la cohésion globale de l'édifice cristallin est assurée par un système de liaisons hydrogène. On retrouve donc dans ces cristaux les caractéristiques favorables de stabilité et de conductibilité thermiques du KDP. L'anion (H₂PO₄⁻)ₙ a une stabilité comparable à celle des cations organiques qui lui sont associés.

Les sels de l'invention peuvent être préparés par un procédé simple consistant à faire réagir une 2-amino-5-nitropyridine de formule : 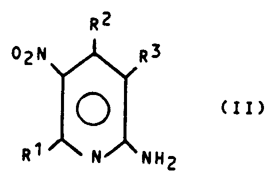
dans laquelle R¹, R² et R³ ont la signification donnée ci-dessus, avec l'acide de formule A⁻H⁺ dans laquelle A⁻ a la signification donnée ci-dessus.

Pour mettre en oeuvre ce procédé, on opère généralement en solution aqueuse en utilisant un excès de l'acide A⁻H⁺, par exemple 3mol d'acide par mol de 2-amino-5-nitropyridine car l'excès d'acide est favorable à la dissolution dans l'eau de la pyridine.

Comme la réaction a lieu dans l'eau, il y a une compétition entre la basicité de l'eau avec formation de H₃O⁺ et celle de la 2-amino-5-nitropyridine qui est une base faible par excellence.

On peut aussi préparer directement le sel à l'abri de l'air sans excès d'eau ou dans l'air en chassant l'eau.

Lorsqu'on veut préparer le sel partiellement ou totalement deutéré, on soumet ensuite le sel obtenu précédemment à une deutération.

L'opération de deutération peut être effectuée par des méthodes classiques d'échange isotopique, par exemple par échange avec de l'eau lourde D₂O, par réaction avec DCl et AlCl₃, par traitement prolongé en présence de D₂SO₄ ou par traitement dans l'ammoniac liquide deutéré en présence de sodium comme catalyseur. Ces méthodes sont décrites dans "Isotopic Exchange and The Replacement of hydrogen in Organic Compounds" (traduit du russe)A.I. Shatenshtein Consultant Bureau N.Y. (1962)- Appendix p. 295-308.

On peut aussi obtenir les sels deutérés par synthèse en faisant réagir une 2-amino-5-nitropyridine de formule (II) partiellement ou totalement deutérée avec l'acide de formule A⁻H⁺ éventuellement deutéré.

La 2-amino-5-nitropyridine deutérée peut être préparée à partir de 2-amino-5-nitropyridine de formule (II) par les procédés de deutération décrits ci-dessus.

La 2-amino-5-nitropyridine de formule (II) peut être préparée par des procédés classiques.

Les sels de 2-amino-5-nitropyridinium de l'invention peuvent être mis facilement sous la forme de monocristaux par croissance cristalline en solution aqueuse d'acide phosphorique en utilisant par exemple l'un des procédés décrits ci-dessous.
- Un procédé utilisant un réacteur à un seul compartiment selon lequel on plonge un germe cristallin du sel dans un cristallisoir, attaché à une tige de verre en rotation, dans une solution saturée du sel dans de l'acide phosphorique. On maintient constante la température du cristallisoir et on controle le degré hygrométrique de l'atmosphère au dessus du cristallisoir pour que l'évaporation ne soit ni trop rapide, ni trop lente. On peut abaisser le température pour diminuer la solubilité quand la croissance cristalline se ralentit. On sort le cristal obtenu et on le replonge dans un cristallisoir dont la solution a les mêmes caractéristiques que la solution de départ jusqu'à ce que la taille optimale soit atteinte.
- Un procédé utilisant un réacteur en verre à double compartiment selon lequel on fait tourner un cristal du sel dans un compartiment du réacteur contenant une solution saturée du sel et on enrichit cette solution à mesure que le cristal croît aux dépens d'une solution saturée qui provient de l'autre compartiment, maintenu à une température plus élevée dans lequel on dissout en permanence une poudre cristalline du sel. La solution circule d'un compartiment à l'autre sous l'effet d'un gradient thermique qui maintient un gradient de concentration.

Les sels optiquement purs de l'invention peuvent trouver de nombreuses applications dans le domaine de l'optique non-linéaire quadratique et des effets correspondants. Ainsi, ils peuvent être utilisés dans des dispositifs pour :
- la génération de second-harmonique (SHG),
- l'addition de fréquences (ou "up-conversion"),
- l'émission paramétrique optique,
- l'amplification paramétrique optique,
- l'oscillation paramétrique optique (OPO),
- la modulation électro-optique ou effet Pockels,
- la conversion de fréquences infra-rouges en fréquences visibles.

Dans ce dernier cas, la nature précise du dispositif et son domaine d'applications dépendront des fenêtres de transparence du sel. C'est ainsi que les sels transparents dans le visible et jusqu'à 400nm conviendront à la conversion vers le bleu de diodes laser émettant vers 850nm, l'application principale étant la réalisation de sources pour mémoires optiques à forte densité. Pour des applications telles que la détection et la mesure des caractéristiques temporelles d'impulsions lumineuses brèves dans le proche infrarouge (1,5µm)par autocorrélation, une transparence plus réduite telle que celle des analogues de la paranitroaniline pourra convenir.

On peut encore utiliser les sels de l'invention pour :
- la détection et la résolution temporelle d'impulsions brèves par autocorrélation (génération de fréquences) ou "up-conversion" (somme de fréquences),
- la réalisation de sources cohérentes accordables dans le proche infrarouge et l'amplification de leurs rayonnements,
- les modulateurs à large bande pour les télécommunications ou le traitement optique du signal (coupleur directif, point de commutation optique), et
- la génération d'états comprimés de lumière ("squeezed states") à caractéristiques de bruit optimal.

On peut aussi utiliser les sels de l'invention pour leurs propriétés ferroélectriques, piézoélectriques et pyroélectriques dans de nombreux dispositifs tels que des mémoires, des capteurs, et des transducteurs agissant sous l'effet de la température, de la pression, d'une tension électrique ou d'une irradiation optique.

Pour les applications en optique non-linéaire, les sels de l'invention à l'état optiquement pur peuvent être utilisés sous différentes formes, par exemple sous la forme de poudres, sous la forme d'inclusions de molécules dans un réseau hôte (polymère, clathrate, solution solide), sous la forme de couches minces déposées sur un substrat, sous la forme de monocristal, sous la forme de solutions.

Généralement, ils sont tout d'abord purifiés, puis mis sous la forme voulue par exemple sous la forme de monocristaux par des procédés classiques, par exemple par évaporation de solvant ou par une méthode de croissance en solution par refroidissement contrôlé. Lorsqu'on veut les utiliser sous la forme de couches minces, on peut préparer de telles couches par la méthode de Langmuir-Blodgett ou encore par épitaxie.

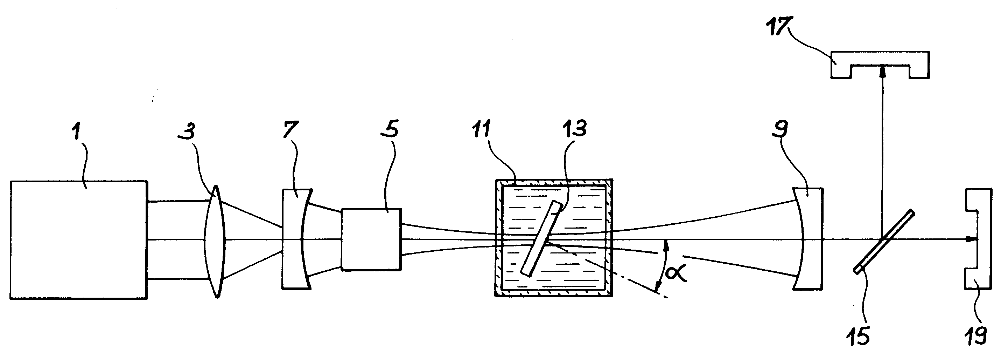

D'autres caractéristiques et avantages de l'invention apparaîtront mieux à la lecture de la description qui suit, d'un exemple de réalisation de l'invention donné bien entendu à titre illustratif et non limitatif, en référence au dessin annexé qui représente de façon schématique un dispositif doubleur de fréquence utilisant un cristal de sel conforme à l'invention.

Cet exemple concerne la préparation et l'utilisation du monophosphate diacide de 2-amino-5-nitropyridinium (2A5NPDP), soit du sel de formule (Ia).

On dissout dans 50cm³ d'une solution aqueuse contenant 3mol d'acide phosphorique, 1mol de 2-amino-5-nitropyridine, à la température de 60°C. On obtient ainsi en solution le monophosphate diacide de 2-amino-5-nitropyridinium (2A5NPDP).

On prépare ensuite à partir de cette solution des cristaux du sel par évaporation à l'air, ce qui donne de beaux cristaux de 2A5NPDP de 5 à 12mm de long et de 4mm de large, de couleur légèrement jaune.

On peut aussi obtenir des cristaux blancs très fins et très purs de 2A5NPDP par précipitation d'une solution saturée de 2A5NPDP, à l'équilibre, par l'alcool éthylique. On obtient ainsi une poudre blanche qui ne se dissout pas dans l'eau mais dans une solution acide à 60°C contenant au moins 2mol de H₃PO₄ pour 1mol du monophosphate diacide de 2-amino-5-nitropyridinium (2A5NPDP). Le 2A5NPDP est facilement décomposé par l'eau chaude en H₃PO₄ et en 2-amino-5-nitropyridine.

On détermine la structure cristalline de la poudre par cristallographie aux rayons X et on observe que la poudre a une structure cristalline polaire (groupe spatial Pna2₁, groupe ponctuel mm₂), et est compatible à la fois avec les exigences de l'optique non-linéaire (effets quadratiques) et de la ferroélectricité.

On effectue sur la poudre du sel obtenu précédemment le test standard de génération de second-harmonique selon la méthode décrite par : S.K. Kurtz et T.T. Perry dans J. Appl. Phys., 39, p. 37-98, (1968), en utilisant une poudre de 2A5NPDP à grains calibrés entre 100 et 200µm de rayon moyen après tamisages successifs, un laser YAG à 1,06µm et l'harmonique à 530nm. Dans ces conditions, la poudre de monophosphate diacide de 2-amino-5-nitropyridinium de l'invention émet un rayonnement vert avec une efficacité de l'ordre de celle de la 3-méthyl-4-nitropyridine-1-oxyde (POM) (légèrement inférieure) et de l'ordre de 10 fois celle de l'urée.

Ainsi, le 2A5NPDP présente une réponse non-linéaire intermédiaire entre celle de l'urée et du POM qui sont des cristaux organiques très utilisés en optique non-linéaire en raison de leur bon compromis entre la transparence et l'efficacité (centrés sur le proche U.V. pour l'urée et sur le proche infra-rouge pour le POM).

La transparence des cristaux de monophosphate diacide de 2-amino-5-nitropyridinium (2A5NPDP) de l'invention est intermédiaire entre celle de l'urée et du POM avec une limite d'absorption à l'état solide située vers 300nm (200nm pour l'urée et 460nm pour le POM).

Les cristaux ont ainsi un domaine de transparence intermédiaire entre celui du composé purement minéral KDP et celui de monocristaux constitués d'unités moléculaires analogues à la paranitroaniline.

L'efficacité de conversion du rayonnement fondamental en rayonnement harmonique bénéficie de l'apport d'un double réseau d'oscillateurs anharmoniques en interaction : l'un attaché au cation organique 2-amino-5-nitropyridinium, l'autre à l'anion minéral H₂PO₄⁻.

A titre d'exemple, on décrit, ci-après, l'utilisation de ce cristal de monophosphate diacide de 2-amino-5-nitropyridinium dans un doubleur de fréquence intracavité similaire à celui décrit par Ducharme et al dans Appl. Phys. Lett. 57(6), 1990, p. 537-539.

Un tel dispositif doubleur de fréquence est représenté schématiquement sur la figure annexée et comprend un laser titane-saphir émettant vers 900 nm, c'est-à-dire dans le domaine de transparence du 2A5NPDP, et un cristal de 2A5NPDP placé dans la cavité interne de ce laser.

Sur la figure annexée, on voit que le dispositif comprend un laser de pompage (1), une lentille (3) pour focaliser le faisceau du laser sur un élément en titane-saphir (5) disposé entre un réflecteur (7) et un coupleur de sortie (9) comportant chacun un revêtement pour assurer respectivement une réflexion de 100% et de 99,5% à la longueur d'onde de 450nm. L'élément (5) en titane-saphir est pourvu d'un revêtement anti-réflexion pour la même longueur d'onde. Une cuvette (11) munie de revêtements anti-réflexion pour les longueurs d'onde de 900nm et 450nm, remplie d'un liquide inerte et non corrosif (par exemple de type fréon)ayant un indice de réfraction permettant une certaine adaptation optique (indice de l'ordre de 1,5) et contenant un cristal (13) de 2 A5NPDP orienté à l'angle d'accord de phase α est disposée entre l'élément (5) et le coupleur de sortie (9).

Un miroir dichroïque (15) placé derrière le coupleur de sortie (9) permet de séparer le faisceau de sortie du laser pour diriger le faisceau fondamental (900nm) vers le premier détecteur (17) et le second harmonique (450nm) vers le second détecteur (19).

## Revendications

1. Sels de 2-amino-5-nitropyridinium répondant à la formule : 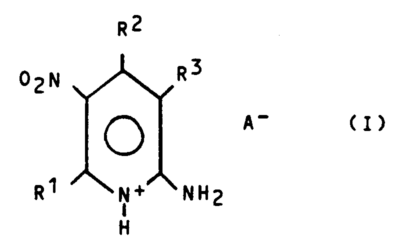 dans laquelle A⁻ représente un anion inorganique choisi parmi H₂PO₄⁻, HSO₄⁻ et H₂AsO₄⁻, et R¹ à R³ qui peuvent être identiques ou différents, représentent H ou CH₃.

2. Sels de 2-amino-5-nitropyridinium deutéré répondant à la formule : 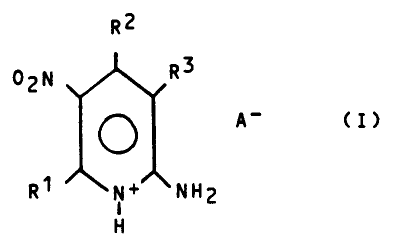 dans laquelle A⁻ représente un anion choisi parmi H₂PO₄⁻, HSO₄⁻ et H₂ASO₄⁻, R¹ à R³ qui peuvent être identiques ou différents, représentent H ou CH₃, et dans laquelle l'un au moins des atomes d'hydrogène est remplacé par un atome de deutérium.

3. Sel de 2-amino-5-nitropyridinium selon la revendication 1 ou 2 répondant à la formule :

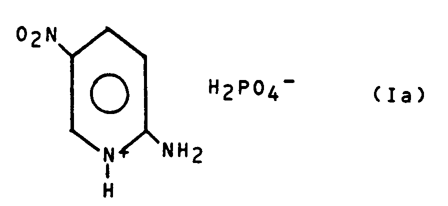

4. Procédé de préparation d'un sel de 2-amino-5-nitropyridinium répondant à la formule : 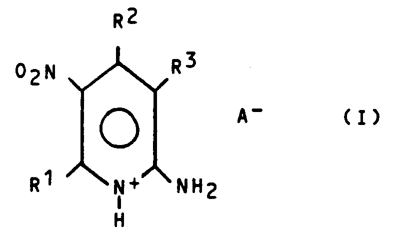 dans laquelle A⁻ représente un anion inorganique choisi parmi H₂PO₄⁻, HSO₄⁻ et H₂AsO₄⁻, et R¹ à R³ qui peuvent être identiques ou différents, représentent H ou CH₃, caractérisé en ce qu'il consiste à faire réagir une 2-amino-5-nitropyridine de formule : 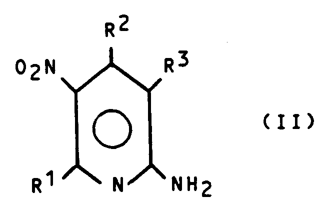 dans laquelle R¹, R² et R³ ont la signification donnée ci-dessus, avec l'acide de formule A⁻H⁺ dans laquelle A⁻ a la signification donnée ci-dessus.

5. Procédé de préparation d'un sel de 2-amino-5-nitropyridinium deutéré de formule : 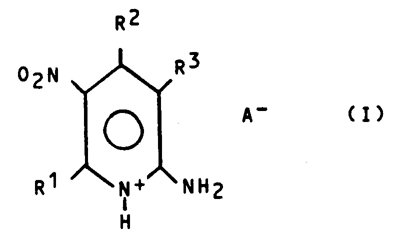 dans laquelle A⁻ représente un anion choisi parmi H₂PO₄⁻, HSO₄⁻ et H₂ASO₄⁻, R¹ à R³ qui peuvent être identiques ou différents, représentent H ou CH₃, et dans laquelle l'un au moins des atomes d'hydrogène est remplacé par un atome de deutérium, caractérisé en ce qu'il consiste à faire réagir une 2-amino-5-nitropyridine de formule 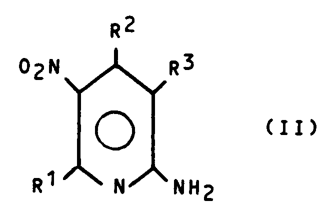 dans laquelle R¹, R² et R³ ont la signification donnée ci-dessus avec un acide de formule A⁻H⁺ dans laquelle A⁻ a la signification donnée ci-dessus, et à deutérer le sel de 2-amino-5-nitropyridinium ainsi obtenu.

6. Utilisation d'un sel de 2-amino-5-nitropyridinium selon l'une quelconque des revendications 1 à 3 dans des dispositifs optiques ou opto-électriques.

## Claims

1. 2-Amino-5-nitropyridinium salts having the formula: 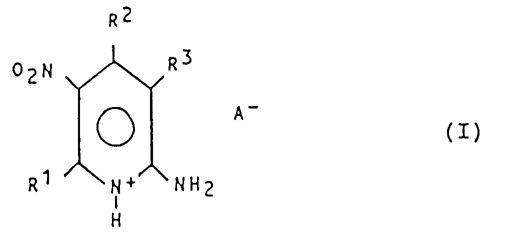 in which A⁻ represents an inorganic anion selected from the group consisting of H₂PO₄⁻, HSO₄⁻ and H₂AsO₄⁻, and R¹ to R³, which can be identical or different from each other, represent H or CH₃.

2. Deuterated 2-amino-5-nitropyridinium salts having the formula: 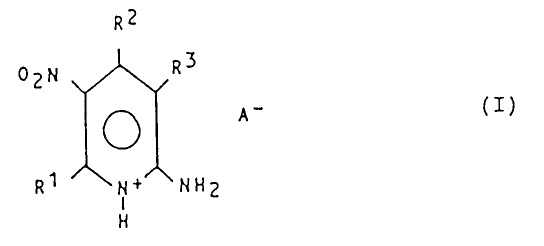 in which A⁻ represents all anion selected from the group consisting of H₂PO₄⁻, HSO₄⁻ and H₂AsO₄⁻, and R¹ to R³, which can be identical or different from each other, represent H or CH₃, and in which at least one of the hydrogen atoms is replaced by a deuterium atom.

3. 2-Amino-5-nitropyridinium salt according to claim 1 or 2, having the formula:

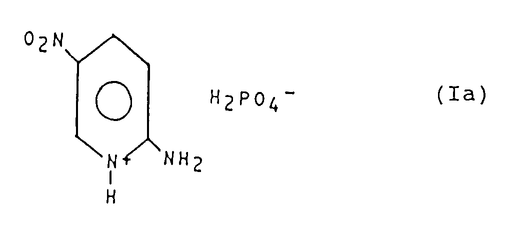

4. Process for preparing a 2-amino-5-nitropyridinium salt of the formula: 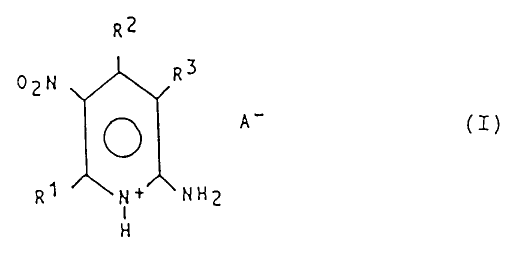 in which A⁻ represents an inorganic anion selected from the group consisting of H₂PO₄⁻, HSO₄⁻ and H₂AsO₄⁻, and R¹ to R³, which can be identical or different from each other, represent H or CH₃, characterized by reacting a 2-amino-5-nitropyridine of the formula: 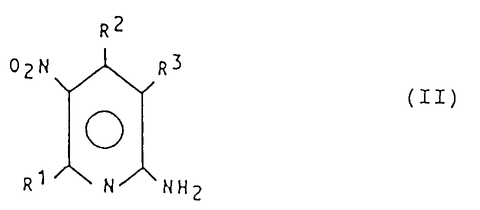 in which R¹, R² and R³ have the meaning given above, with an acid of formula A⁻H⁺ in which A⁻ has the meaning given above.

5. Process for preparing a deuterated 2-amino-5-nitropyridinium salt of the formula: 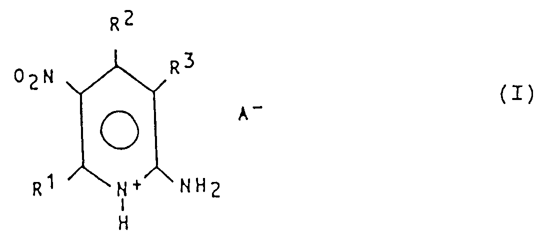 in which A⁻ represents an anion selected from the group consisting of H₂PO₄⁻, HSO₄⁻ and H₂AsO₄⁻, and R¹ to R³, which can be identical or different from each other, represent H or CH₃, and in which at least one of the hydrogen atoms is replaced by a deuterium atom, characterized by reacting a 2-amino-5-nitropyridine of the formula: 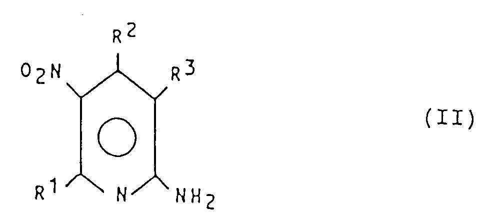 in which R¹, R² and R³ have the meaning given above, with an acid of formula A⁻H⁺ in which A⁻ has the meaning given above, and in deuterating the thus obtained 2-amino-5-nitropyridinium salt.

6. Use of a 2-amino-5-nitropyridinium salt according to any one of the claims 1 to 3 in optical or opto-electrical devices.

## Patentansprüche

1. 2-Amino-5-nitropyridiniumsalze der Formel 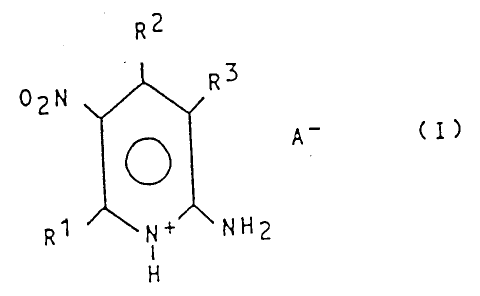 worin A⁻ ein anorganisches Anion, ausgewählt aus H₂PO₄⁻, HSO₄⁻ und H₂AsO₄⁻, darstellt und R¹ bis R³, die gleich oder verschieden sein können, H oder CH₃ darstellen.

2. Deuterierte 2-Amino-5-nitropyridiniumsalze der Formel 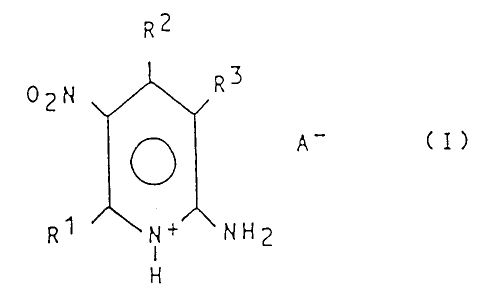 worin A⁻ ein Anion, ausgewählt aus H₂PO₄⁻, HSO₄⁻ und H₂AsO₄⁻, darstellt, R¹ bis R³, die gleich oder verschieden sein können, H oder CH₃ darstellen und worin wenigstens eins der Wasserstoffatome durch ein Deuteriumatom ersetzt ist.

3. 2-Amino-5-nitropyridiniumsalz nach Anspruch 1 oder 2 der Formel

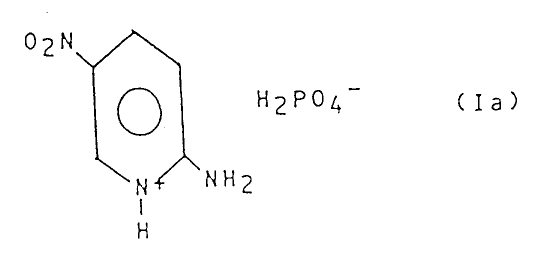

4. Verfahren zur Herstellung eines 2-Amino-5-nitropyridiniumsalzes der Formel 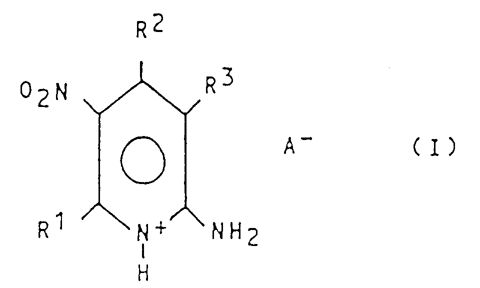 worin A⁻ ein anorganisches Anion, ausgewählt aus H₂PO₄⁻, HSO₄⁻ und H₂AsO₄⁻, darstellt und R¹ bis R³, die gleich oder verschieden sein können, H oder CH₃ darstellen, dadurch gekennzeichnet, daß es in der Reaktion eines 2-Amino-5-nitropyridins der Formel 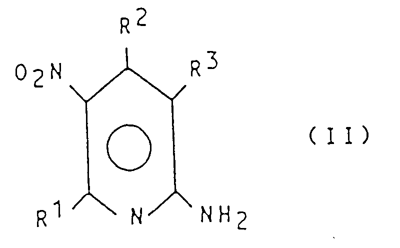 worin R¹, R² und R³ die oben gegebene Bedeutung aufweisen, mit einer Säure der Formel A⁻H⁺, worin A⁻ die oben gegebene Bedeutung aufweist, besteht.

5. Verfahren zur Herstellung eines deuterierten 2-Amino-5-nitropyridiniumsalzes der Formel 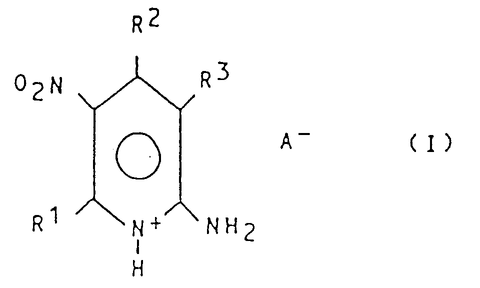 worin A⁻ ein Anion, ausgewählt aus H₂PO₄⁻, HSO₄⁻ und H₂AsO₄⁻, darstellt, R¹ bis R³, die gleich oder verschieden sein können, H oder CH₃ darstellen, und worin wenigstens eines der Wasserstoffatome durch ein Deuteriumatom ersetzt ist, dadurch gekennzeichnet, daß es in der Reaktion eines 2-Amino-5-nitropyridins der Formel 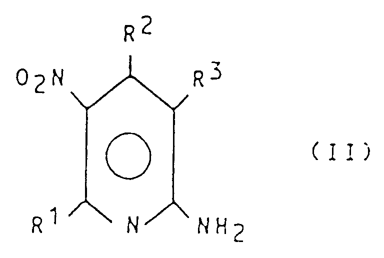 worin R¹, R² und R³ die oben gegebene Bedeutung aufweisen, mit einer Säure der Formel A⁻H⁺, worin A⁻ die oben gegebene Bedeutung hat, und der Deuterierung des so erhaltenen 2-Amino-5-nitropyridiniumsalzes besteht.

6. Verwendung eines 2-Amino-5-nitorpyridiniumsalzes nach einem der Ansprüche 1 bis 3 in optischen oder opto-elektrischen Vorrichtungen.
